# EUROPEAN PATENT SPECIFICATION

(11) **EP 2 868 335 B1**
(45) Date of publication and mention of the grant of the patent: **10.04.2019**
(21) Application number: 14382434.0
(22) Date of filing: 04.11.2014
(51) Int. Cl.: A61B 10/00, A61B 10/02, A61B 17/00, A61M 1/00, A61B 17/34, A61M 39/02, A61M 25/06

(54) **Device to drain an abscess**
Vorrichtung zur Ableitung eines Abszesses
Dispositif pour drainer un abcès

(30) Priority: 05.11.2013 ES 201331265 U
(43) Date of publication of application: 06.05.2015
(73) Proprietor: Teruelo Prieto, Javier, 01010 Vitoria (ES); Robles Fernandez, Ruben, 01010 Vitoria (ES); Sanchez Oliver, Iker, 01010 Vitoria (ES); Martin Torrecilla, Miguel, 01010 Vitoria (ES)
(72) Inventor: Teruelo Prieto, Javier, 01010 Vitoria (ES)
(74) Representative: Pons

(56) References cited:
- EP-A1- 0 260 543
- WO-A1-2008/029109
- GB-A- 1 434 507
- GB-A- 2 185 689
- GB-A- 2 344 289

## Description

### OBJECT OF THE INVENTION

The present invention can be included in the technical field of medical and surgical instruments. In particular, the invention has the object of a device for draining an abscess.

### BACKGROUND OF THE INVENTION

A perirectal abscess or, where applicable, anorectal abscess, is an ailment wherein an infection occurs in the tissues surrounding the rectum or, where applicable, the anus. The infection results in the formation of a sac filled with pus inside the affected tissue. It is also possible that a duct forms, called fistula, which runs from the abscess towards other surrounding tissues, or even towards the rectum, or where applicable, the anus.

The abscess causes symptoms such as pain, inflammation, fever, etc. until the source of the infection is eliminated.

To treat the abscess, normally two cooperative paths are followed: the causes of the infection are treated and, in parallel, the pus is drained to avoid the infection spreading to other tissues and therefore leading to more serious complications. The drainage is traditionally performed by means of a surgical intervention wherein an incision is made to remove the pus, generally using local anaesthesia. On some occasions, the patient must keep in place a drainage element, of "penrose" type during several days, once discharged after the intervention.

United Kingdom patent application GB2185689 discloses an apparatus for draining fluid from a body cavity, comprising a housing having an integral cannula in permanent fluid communication with an outlet port, and an access port closed by a self-sealing elastomeric plug through which passes a removable needle. A tube attached to the outlet port can include a double check valve and pump port to permit aspiration of fluid from the cavity. The apparatus may include a flange and a locking device on the cannula to fix the apparatus to the patient's body at a desired insertion depth.

Also, United Kingdom patent application GB2344289 shows a hollow needle sheathed by a cannula for use in the treatment of pneumothorax (collapsed lung), incorporating a signalling device to show correct positioning of the needle tip. The signalling device comprising a housing that communicates with the interior of the hollow needle and has an opening 4 remote from the needle, the housing containing a member movable in response to breathing. The hollow needle may be sheathed in a cannula part that is sealable at one end and open at the other, and may have means along its length for attachment to the chest wall and for a portal for the controlled passage of a gas or liquid. The purpose of the whole is to simplify the treatment of pneumothorax by providing a dedicated piece of equipment for that process.

International application WO2008/029109 is related to an apparatus for treating pneumothorax comprises a catheter and a needle with a pointed tip removably received in the catheter. The catheter has a planar mount with an adhesive surface or suture boles by which the catheter can be secured to the patient. A thumb pad at the upper end of the needle enables pressure to be applied during insertion. The apparatus may include a visual or audible indicator to indicate correct placement of the apparatus. A one-way valve may be attached with the catheter after removal of the needle to allow air lo escape from the pleural cavity without allowing air to flow into the cavity via the apparatus.

United States patent US1434507 shows an apparatus for suprapubic catherization comprising a hollow hub, a flexible catheter tube extending from and communicating with the hub and having at least one opening at the end thereof remote from the hub, a hollow elongated stylet which has an external diameter slightly less than the internal diameter of the catheter tube, has a pointed closed tip and an opening close to the tip and can be positioned within the catheter tube through the hub with its tip projecting from the tube, a flexible drainage tube which is attached or attachable to the hub to enable body fluid entering the apparatus through the openings in the stylet and the catheter tube to be drained from the hub, and a locking mechanism which is slidable on the catheter tube and includes a male part having a tapered portion which fits tightly into a tapered recess in a female part, the male part having at least one slit in its tapered portion.

European patent application EP0260543 discloses an apparatus for drainage of a body cavity, including a housing body defining an inlet chamber and a discharge chamber, with the former having a catheter mounted to one wall thereof and communicating with the inlet chamber, and a one-way elongated valve in fluid communication between the inlet chamber and the discharge chamber. An inlet port is provided in another wall of the housing body defining the inlet chamber which is adapted to reversibly receive a trocar and to enable at least a portion of the trocar to pass through the inlet chamber and the catheter to enable the insertion of the catheter into the body cavity to be drained. A hydrophobic filter is provided along one wall of the discharge chamber to filter gaseous fluids therefrom. Discharge and suction means can also be provided to the discharge chamber.

### DESCRIPTION OF THE INVENTION

The present invention has the object of a device for draining an abscess, which makes it possible to drain the abscess of a patient to mitigate the aforementioned symptoms whilst the treatment aimed at eradicating the source of the infection which gives rise to the abscess or the fistula lasts. Through the use of the device for draining an abscess of the present invention, as shall be described below, it is possible to drain an abscess without surgical intervention, i.e. in a less invasive way than those shown in the state of the art.

The device of the invention is based on a combination of a cannula and a needle housed in the cannula. A proximal end of the needle, at the same time as a proximal end of the cannula, are introduced in the patient's body in a tissue affected by an abscess, until the mentioned respective proximal ends of the cannula and of the needle access a sac of pus of the abscess. Then, the needle is separated from the cannula, longitudinally displacing the needle throughout the cannula until extracting the proximal end of the needle through the distal end of the cannula.

An adhesive flap located in the distal end of the cannula, and protruding from the cannula towards the outside makes it possible to hold the cannula in the patient's body. The pus shall be accessed from the sac of pus of the interior of the abscess towards the outside of the patient's body through the distal end of the cannula.

### DESCRIPTION OF THE DRAWINGS

To complement the description being made and in order to aid towards the understanding of the characteristics of the invention, in accordance with a preferred example of practical embodiment thereof, a set of drawings is attached to the present specification wherein, with illustrative and non-limitative character, the following has been represented:
Figure 1.- Shows a sectional view of a first embodiment of the device of the invention.
Figure 2.- Shows a sectional view of a second embodiment of the device of the invention.
Figure 3.- Shows a detailed perspective view of the flap.

### PREFERRED EMBODIMENT OF THE INVENTION

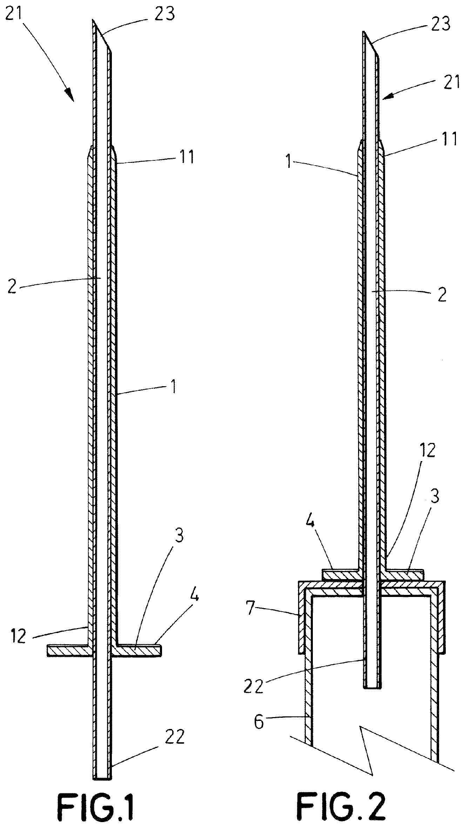

A detailed description of a preferred embodiment of the invention is described below, with the aid of aforementioned figures 1 to 3.

The device for draining an abscess according to the present invention comprises, as observed in figures 1 and 2, a cannula (1) equipped with:
- A proximal end (11) designed to be inserted inside the body of a patient in a tissue affected by an abscess; and
- A distal end (12) designed to remain inside the patient's body.

The device of the invention further comprises, as shown in figures 1 and 2, a needle (2) equipped with:
- A proximal end (21);
- a point (23), located at the proximal end (21), and designed to be inserted, together with the proximal end (21) of the needle (2), inside the abscess until reaching a sac of pus; and
- A distal end (22), designed to remain inside the patient's body.

The needle (2) is configured so as to be longitudinally housed inside the cannula (1), according to a first position wherein the point (23) of the needle (2) protrudes from the proximal end (11) of the cannula (1). Additionally, the needle (2) is separable from the cannula (1), by extraction of the needle (2) through the distal end (12) of the cannula (1); i.e. the needle (2) is displaceable inside the cannula (1) to a second position wherein the needle (2) is outside the cannula (1).

The device additionally incorporates, in the distal end (12) of the cannula (1), a flap (3), preferably of deformable material, for fixing the cannula (1) to the patient's body, when the proximal end (11) of the cannula (1) is in contact with the sac of pus. The flap (3) has a first through-bore (not shown) for connecting the cannula (1) with the flap (3). The flap (3) may have, for example, oval, polygonal, circular form, etc. The flap (3) protrudes towards the outside from the cannula (1), and the flap (3) is impregnated with an adhesive product (4) to allow the fixing of the flap (3) to the patient's body. The flap (3) is integrally configured with the cannula (1).

Figure 1 shows a first embodiment wherein the device of the invention only comprises:
- The cannula (1),
- The needle (2) and
- The flap (3), impregnated with adhesive product (4), as well as integrally configured with the cannula (1), defining a flap (3)-cannula (1) assembly.

Preferably, according to another embodiment of the invention, the device additionally incorporates a vacuum tube (6) coupled in the distal end (22) of the needle (2) and/or in the distal end (12) of the cannula. Preferably, the vacuum tube (6) can be coupled through a corresponding tube holder (7). The vacuum tube (6) may be coupled to the cannula (1) and/or the needle (2) removably. However, figure 2 shows an example wherein the tube holder (7) is integrally configured with the distal end (22) of the needle (2), defining a needle (2) plus tube holder (7) assembly.

To perform a drainage of an abscess using the device of the invention, one starts from the first position, wherein the point (23) of the needle (2) is visible, protruding from the proximal end (11) of the cannula (1), and the point (23) of the needle (2) is inserted into the patient's body, in a tissue affected by an abscess, until reaching a sac of pus. Simultaneously, the cannula (1) is introduced together with the needle (2), from the proximal end (11). The proximal end (11) of the cannula may incorporate a chamfer, which may have an inclination of approximately 15°, to facilitate the insertion of the cannula (1) in the patient's body.

When the respective proximal ends (11, 21) of the cannula (1) and the needle (2) have reached the sac of pus, the needle (2) is separated from the cannula (1), holding the cannula (1) and displacing the needle (2) longitudinally towards the outside of the patient's body, towards the second position, so that the proximal end (11) of the cannula (1) remains in the sac of pus and the distal end (12) of the cannula (1) is found in the exterior of the patient. The cannula (1) remains fixed to the patient's body by the fixing means, for example, the deformable adhesive flap (3) represented in figures 1 and 2.

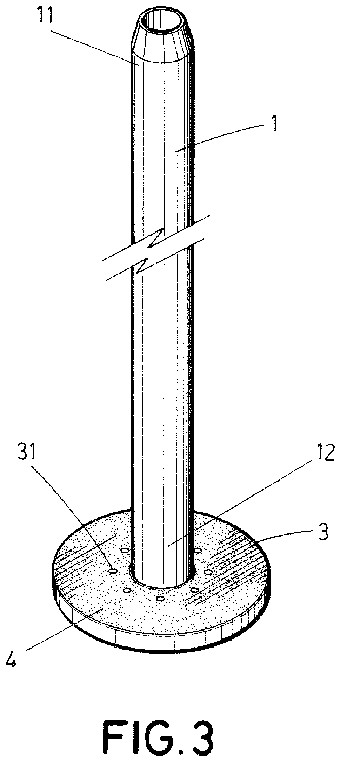

The adhesive flap (3) can incorporate at least a second through-bore (31), externally to the cannula (1), as shown in figure 3, to allow remains of pus and/or of blood, which eventually escape through the outside of the cannula (1), traversing the flap (3), towards the outside of the patient. Therefore, it avoids the adhesive product (4) from becoming soaked in pus and/or blood and losing its adhesive capacity.

The incorporation of the vacuum tube (6) makes it possible to optimize the use of the device of the invention, in the sense that, on the one hand, the suitable moment when the needle (2) should be removed is indicated cleanly by the arrival of the pus to the vacuum vessel (6) through the needle (2). Additionally, the vacuum in the vacuum vessel (6) causes a depression which accelerates the arrival of pus to the vacuum vessel (6), through the needle (2), so that it also accelerates the drainage process. Additionally, the vacuum vessel (6) makes it possible to cleanly and quickly collect samples of pulse to perform analyses.

During a time, which may last several days, the pus will exit the sac of pus towards the outside of the patient through the cannula (1). With the aim that the patient does not dirty their clothes, a pad or gauze can be placed to absorb said pus.

Since the flap (3) is designed to be located at an end distal position in contact with the patient's body, the use of the device of the invention is more discrete, less uncomfortable and safer than the use of a penrose-type device.

## Claims

1. Device for draining an abscess, **characterized in that** it comprises:
- a cannula (1), equipped with:
- a proximal end (11) designed to be inserted inside the body of a patient in a tissue affected by the abscess; and
- a distal end (12) designed to remain inside the patient's body;
- a needle (2), equipped with:
- a distal end (22), designed to remain inside the patient's body,
- a proximal end (21), and
- a point (23), located in the proximal end (21) of the needle (2), and designed to be inserted inside the abscess until reaching a sac of pus;
wherein the needle (2) is configured so as to be longitudinally housed inside the cannula (1), according to a first position wherein the point (23) protrudes from the proximal end (11) of the cannula (1); as well as the needle (2) can be displaced inside the cannula (1) to a second position wherein the needle (2) is outside the cannula (1), for separating the needle (2) and the cannula (1) from one another; and
- a flap (3), located in the distal end (12) of the cannula (1) and protruding from the cannula (1) towards the outside, the flap (3) comprising:
- a first through-bore to connect the flap (2) to the cannula (1); and
- an adhesive product (4), for fixing the cannula (1) to the patient's body, so as to allow the patient to carry the device out of a health center.

2. Device for draining an abscess, according to claim 1, wherein the flap (3) is integrally configured with the cannula (1).

3. Device for draining an abscess, according to any of claims 1 and 2, wherein the flap (3) incorporates at least one second bore (31) external to the first through-bore.

4. Device for draining an abscess, according to claim 1, wherein the flap (3) is deformable.

5. Device for draining an abscess, according to claim 1, wherein it additionally incorporates a vacuum tube (6) coupled in the distal end (22) of the needle (2) and/or in the distal end (12) of the cannula.

6. Device for draining an abscess, according to claim 5, wherein it additionally incorporates a tube holder (7) for coupling the vacuum tube (6).

7. Device for draining an abscess, according to claim 6, wherein the tube holder (7) is integrally configured with the distal end (22) of the needle (2).

## Patentansprüche

1. Vorrichtung zum Drainieren eines Abszesses, **dadurch gekennzeichnet, dass** sie Folgendes umfasst:
- eine Kanüle (1), mit Folgendem ausgestattet:
- ein proximales Ende (11), das dazu ausgelegt ist, in den Körper eines Patienten in einem von dem Abszess betroffenen Gewebe eingeführt zu werden; und
- ein distales Ende (12), das dazu ausgelegt ist, im Körper des Patienten zu verbleiben;
- eine Nadel (2), mit Folgendem ausgestattet:
- ein distales Ende (22), das dazu ausgelegt ist, im Körper des Patienten zu verbleiben,
- ein proximales Ende (21), und
- eine Spitze (23), die sich am proximalen Ende (21) der Nadel (2) befindet und dazu ausgelegt ist, in den Abszess eingeführt zu werden, bis sie einen Eitersack erreicht;
wobei die Nadel (2) so ausgebildet ist, dass sie in Längsrichtung innerhalb der Kanüle (1) gemäß einer ersten Position, in der die Spitze (23) vom proximalen Ende (11) der Kanüle (1) vorsteht, untergebracht ist; wobei die Nadel (2) ferner innerhalb der Kanüle (1) in eine zweite Position verschoben werden kann, in der sich die Nadel (2) außerhalb der Kanüle (1) befindet, um die Nadel (2) und die Kanüle (1) voneinander zu trennen; und
- einen Lappen (3), der sich im distalen Ende (12) der Kanüle (1) befindet und von der Kanüle (1) nach außen vorsteht, wobei der Lappen (3) Folgendes umfasst:
- eine erste Durchgangsbohrung, um den Lappen (2) mit der Kanüle (1) zu verbinden; und
- ein Klebeprodukt (4), um die Kanüle (1) am Körper des Patienten zu befestigen, so dass der Patient die Vorrichtung aus einem Gesundheitszentrum heraus mitführen kann.

2. Vorrichtung zum Drainieren eines Abszesses nach Anspruch 1, wobei der Lappen (3) integral mit der Kanüle (1) ausgebildet ist.

3. Vorrichtung zum Drainieren eines Abszesses nach einem der Ansprüche 1 und 2, wobei die Klappe (3) mindestens eine zweite Bohrung (31) außerhalb der ersten Durchgangsbohrung aufweist.

4. Vorrichtung zum Drainieren eines Abszesses nach Anspruch 1, wobei die Klappe (3) verformbar ist.

5. Vorrichtung zum Drainieren eines Abszesses nach Anspruch 1, wobei sie zusätzlich eine Vakuumröhre (6) beinhaltet, die am distalen Ende (22) der Nadel (2) und/oder am distalen Ende (12) der Kanüle gekoppelt ist.

6. Vorrichtung zum Drainieren eines Abszesses nach Anspruch 5, wobei sie zusätzlich einen Rohrhalter (7) zum Koppeln der Vakuumröhre (6) beinhaltet.

7. Vorrichtung zum Drainieren eines Abszesses nach Anspruch 6, wobei der Rohrhalter (7) integral mit dem distalen Ende (22) der Nadel (2) ausgebildet ist.

## Revendications

1. Dispositif pour le drainage d'un abcès, **caractérisé en ce qu'**il comprend :
- une canule (1), équipée de :
- une extrémité proximale (11) conçue pour être insérée dans le corps d'un patient dans un tissu affecté par l'abcès ; et
- une extrémité distale (12) conçue pour rester dans le corps du patient ;
- un aiguille (2), équipée de :
- une extrémité distale (22), conçue pour rester dans le corps du patient,
- une extrémité proximale (21), et
- un point (23), situé dans l'extrémité proximale (21) de l'aiguille (2), et conçu pour être inséré dans l'abcès jusqu'à atteindre un sac de pus ; dans lequel l'aiguille (2) est configurée pour être logée longitudinalement dans la canule (1), selon une première position dans laquelle le point (23) fait saillie de l'extrémité proximale (11) de la canule (1) ; aussi, l'aiguille (2) peut être déplacée dans la canule (1) à une deuxième position dans laquelle l'aiguille (2) est à l'extérieur de la canule (1), pour séparer l'aiguille (2) et la canule (1) l'un de l'autre ; et
- un rabat (3), situé à l'extrémité distale (12) de la canule (1) et faisant saillie de la canule (1) vers l'extérieur, le rabat (3) comprenant :
- un premier trou traversant pour raccorder le rabat (2) à la canule (1) ; et
- un produit adhésif (4), pour fixer la canule (1) au corps du patient, de sorte à permettre au patient d'emporter le dispositif hors d'un centre de santé.

2. Dispositif pour le drainage d'un abcès, selon la revendication 1, dans lequel le rabat (3) est intégralement configuré avec la canule (1).

3. Dispositif pour le drainage d'un abcès, selon l'une quelconque des revendications 1 et 2, dans lequel le rabat (3) incorpore au moins un deuxième trou (31) externe au premier trou traversant.

4. Dispositif pour le drainage d'un abcès, selon la revendication 1, dans lequel le rabat (3) est déformable.

5. Dispositif pour le drainage d'un abcès, selon la revendication 1, dans lequel il incorpore en outre un tube sous vide (6) couplé dans l'extrémité distale (22) de l'aiguille (2) et/ou dans l'extrémité distale (12) de la canule.

6. Dispositif pour le drainage d'un abcès, selon la revendication 5, dans lequel il incorpore en outre un support de tube (7) pour coupler le tube sous vide (6).

7. Dispositif pour le drainage d'un abcès, selon la revendication 6, dans lequel le support du tube (7) est intégralement configuré avec l'extrémité distale (22) de l'aiguille (2).
